# DEMANDE DE BREVET EUROPEEN

(11) **EP 1 847 252 A2**
(43) Date de publication de la demande: **24.10.2007**
(21) Numéro de dépôt: 07105458.9
(22) Date de dépôt: 02.04.2007
(51) Int. Cl.: A61K 8/73, A61K 8/60, A61K 8/37, A61Q 5/06, A61Q 5/12

(54) **Composition comprenant un monomère cyanoacrylate, un composé glucidique et un solvant organique liquide et procédés de traitement cosmétique**

(30) Priorité: 13.04.2006 FR 0603329
(71) Demandeur: L'Oréal, 75008 Paris (FR)
(72) Inventeur: Vic, Gabin, 60280, VENETTE (FR); Livoreil, Aude, 75006, PARIS (FR)
(74) Mandataire: Le Blainvaux Bellegarde, Françoise

(57) **Abrégé**

La présente invention concerne une composition cosmétique comprenant au moins un monomère cyanoacrylate, au moins un composé glucidique et au moins un solvant organique liquide.

L'invention est aussi relative à un procédé de traitement mettant en oeuvre cette composition et à son utilisation pour conférer un effet de conditionnement et/ou un effet coiffant aux fibres kératiniques telles que les cheveux.

## Description

La présente invention est relative à une composition comprenant au moins un monomère cyanoacrylate, au moins un composé glucidique et au moins un solvant organique, à son utilisation pour le traitement cosmétique des fibres kératiniques et à un procédé de traitement cosmétique mettant en oeuvre une telle composition.

Dans le domaine de la cosmétique, on cherche à modifier les propriétés de surface des fibres kératiniques telles que les cheveux, par exemple pour apporter aux cheveux un effet conditionnant comme la douceur, ou de la brillance ou un effet coiffant permettant d'apporter du corps, de la masse ou du volume aux cheveux.

Pour ce faire, on utilise généralement des compositions cosmétiques à base d'agents de conditionnement tels que des silicones ou des polymères ayant une forte affinité pour les fibres kératiniques, et notamment pour les cheveux.

Cependant, ces agents de conditionnement ou de coiffage ont tendance à s'éliminer au cours de lavage avec des shampoings, rendant nécessaire le renouvellement des applications des compositions sur les cheveux.

Pour accroître la rémanence de dépôt de polymères, il peut être envisagé d'effectuer une polymérisation radicalaire de certains monomères directement sur les cheveux. Toutefois, on constate une forte dégradation des fibres capillaires liées probablement aux amorceurs de polymérisation et les cheveux ainsi traités sont difficilement démêlables. On connaît également le document FR 2833 489 qui décrit la polymérisation de monomère électrophile sur les cheveux.

Par ailleurs, ces gainages sont perturbés par le sébum produit par les glandes sébacées.

En effet, les cheveux regraissent très rapidement au cours de temps et les cheveux ont alors un aspect gras et sales.

A la date d'aujourd'hui il n'existe pas de compositions cosmétiques permettant d'apporter du corps, de la masse ou du volume de manière durable à la chevelure, tout en ayant de bonnes propriétés cosmétiques au cours du temps.

La demanderesse vient de découvrir qu'il est possible d'obtenir une amélioration du corps, de la masse ou du volume de la chevelure, une diminution du regraissage des cheveux et ce, de façon durable, en associant au moins un composé glucidique défini plus loin, au moins un monomère cyanoacrylate et au moins un solvant organique.

En effet, l'application d'une composition comprenant une telle association conduit à la formation d'un revêtement ou gainage rémanent notamment aux shampoings, même après plusieurs lavages de la chevelure. Le gainage obtenu peut apporter, en plus des propriétés de coiffage rémanent, des propriétés de brillance et de conditionnement.

L'ajout de polysaccharide diminue fortement l'effet du au sébum. Les cheveux regraissent moins rapidement dans le temps tout en gardant de bonnes propriétés de coiffage.

Les cheveux restent de manière surprenante parfaitement individualisés et peuvent être coiffés sans problème.

Sans que cette explication soit limitative, il semble que ce soit les ions hydroxyde contenus dans l'eau absorbée par les cheveux qui enclenchent le processus de polymérisation anionique à l'interface composition de traitement-cheveux. Le polymère ainsi formé in situ par polymérisation anionique interfaciale se présente sous forme d'un dépôt homogène, lisse et possède une excellente adhésion sur cheveux.

L'invention a donc pour objet une composition cosmétique comprenant :
- au moins un monomère cyanoacrylate,
- au moins un composé glucidique,
- au moins un solvant organique liquide.

Un autre objet de la présente invention consiste en l'utilisation de ladite composition pour le traitement cosmétique des fibres kératiniques telles que les cheveux.

L'invention a encore pour objet un procédé de traitement cosmétique des cheveux, mettant en oeuvre ladite composition.

D'autres objets, caractéristiques, aspects et avantages de l'invention apparaîtront encore plus clairement à la lecture de la description et de l'exemple qui suit.

De préférence, le solvant organique liquide est différent du composé glucidique et du monomère cyanoacrylate.

Les compositions de l'invention comprennent au moins un solvant organique liquide.

Les solvants organiques sont choisis parmi les composés liquides à la température de 25°C et sous 105 Pa (760mm de Hg).

Par solvant organique, on entend une substance capable de dissoudre une autre substance sans la modifier chimiquement.

Le solvant organique est par exemple choisi parmi :
les alcools aromatiques tels que l'alcool benzylique ; les alcools gras liquides , notamment en C₁₀-C₃₀; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques de synthèse ou végétales, les alcanes et plus particulièrement les alcanes de C₅ à C_{10;} les acides gras liquides, les esters gras liquides et plus particulièrement les benzoates ou les salicylates d'alcool gras liquides et leurs mélanges.

Le solvant organique est de préférence choisi parmi les huiles organiques ; les silicones telles que les silicones volatiles, les gommes ou huiles de silicones aminés ou non et leurs mélanges ; les huiles minérales ; les huiles végétales telles que les huiles d'olive, de ricin, de colza, de coprah, de germe de blé, d'amande douce, d'avocat, de macadamia, d'abricot, de carthame, de noix de bancoulier, de camélina, de tamanu, de citron ou encore des composés organiques tels que des alcanes en C₅-C₁₀, l'acétone, la méthyléthylcétone, les esters d'acides en C₁-C₂₀ liquides et d'alcools en C₁-C₈ tels que l'acétate de méthyle, l'acétate de butyle, l'acétate d'éthyle et le myristate d'isopropyle, le diméthoxyéthane, le diéthoxyéthane, les alcools gras liquides en C₁₀-C₃₀ tels que l'alcool oléique, les esters d'alcools gras en C₁₀-C₃₀ liquides tels que les benzoates d'alcool gras en C₁₀-C₃₀ l'isononanoate d'isononyle, le malate d'isostéaryle, le tétra-isostéarate de pentaérythrityle, le trimélate de tridécyle et leurs mélanges ; l'huile de polybutène, le mélange cyclopentasiloxane (14,7% en poids)/polydiméthylsiloxane dihydroxylé en positions alpha et oméga (85,3% en poids), ou leurs mélanges.

Selon un mode de réalisation préféré, le solvant organique est constitué par une silicone ou un mélange de silicones tels que les polydiméthylsiloxanes liquides et les polydiméthylsiloxanes modifiées liquides, la viscosité de la silicone et/ou du mélange de silicones à 25°C est comprise entre 0.1 cst et 1 000 000cst et plus préférentiellement entre 1 cst et 30 000cst.

On citera de préférence les silicones suivantes :
- le mélange de polydiméthylsiloxane alpha-omega-dihydroxylé/cyclopentadiméthylsiloxane (14,7/85,3) commercialisé par Dow Corning sous le nom de DC 1501 Fluid
- le mélange de polydiméthylsiloxane alpha-omega-dihydroxylé/polydiméthylsiloxane commercialisé par Dow Corning sous le nom de DC 1503 Fluid
- le mélange de diméthicone /cyclopentadiméthylsiloxane commercialisé par Dow Corning sous le nom de DC 1411 Fluid ou celui commercialisé par Bayer sous le nom SF1214 ;
- la cyclopentadiméthylsiloxane commercialisée par Dow Corning sous le nom de DC245 Fluid ;

Et les mélanges respectifs de ces huiles.

Les compositions selon l'invention peuvent contenir, outre le ou les solvants organiques liquides, de l'eau. De préférence les compositions sont anhydres c'est-à-dire qu'elles contiennent moins de 1% en poids d'eau par rapport au poids total de la composition.

Le ou les solvants organiques de la composition représentent généralement de 0,01 à 99 %, de préférence de 0,1 à 50 %, en poids par rapport au poids total de la composition.

Le ou les monomères cyanoacrylate présents dans la composition de l'invention sont choisis parmi les monomères de formule **(I) :** 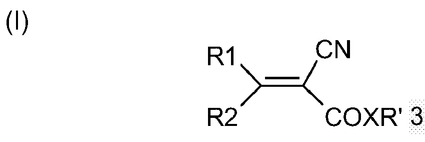 dans laquelle :
- X désigne NH, S ou O,
- R1 et R2 désignent chacun un atome d'hydrogène,

De préférence R1 et R2 représentent un atome d'hydrogène,

R'3 représente un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', - COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀..

De préférence R'3 est un groupe hydrocarboné saturé comportant de 1 à 10 atomes de carbone.

De préférence, X désigne O.

A titre de composés de formule (I), on peut citer les monomères:
a) appartenant à la famille des 2-cyanoacrylate de polyfluoroalkyle tels que:
   l'ester 2,2,3,3-tétrafluoropropylique de l'acide 2-cyano-2-propénoïque de formule : 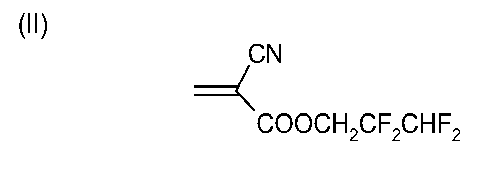
   ou encore l'ester 2,2,2-trifluoroéthylique de l'acide 2-cyano-2-propénoïque de formule : 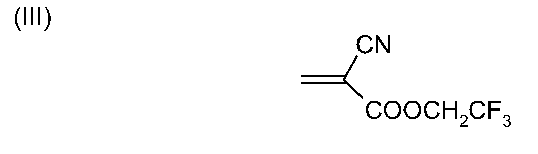
b) les 2-cyanoacrylate d'alkyle ou d'alcoxyalkyle 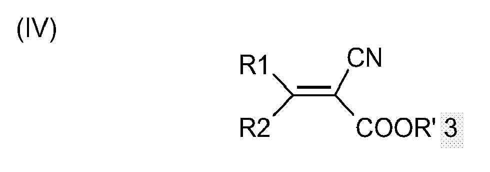 dans laquelle R'3 représente un radical alkyle en C1-C10, un radical alcényle en C2-C10 ou alcoxy(C1-C4) alkyle(C1-C10).

On peut citer plus particulièrement le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de methoxypropyle, le 2-cyanoacrylate de 2-octyle, le 2-cyanoacrylate de 2-propoxyéthyle, le 2-cyanoacrylate de n-octyle le 2-cyanoacrylate d'allyle, le 2-cyanoacrylate de méthoxypropyle et le cyanoacrylate d'iso-amyle.

Dans le cadre de l'invention, on préfère utiliser les monomères b). Selon un mode de réalisation préféré, le ou les monomères cyanoacrylates sont choisis parmi les cyanoacrylates d'alkyle en C6-C10.

Les monomères particulièrement préférés sont les cyanoacrylate d'octyle de formule V et leurs mélanges : 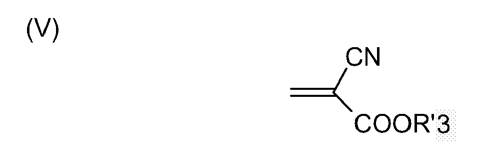 dans laquelle :
- R'3: =-(CH₂)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

Le monomère cyanoacrylate particulièrement préféré selon l'invention est le méthylheptyl 2-cyanoacrylate vendu sous la référence commerciale RITELOK CDN 1064 par la société Chemence.

Les monomères utilisés conformément à l'invention peuvent être fixés de façon covalente sur des supports tels que des polymères, des oligomères ou des dendrimères. Le polymère ou l'oligomère peut être linéaire, ramifié, en peigne ou bloc. La répartition des monomères de l'invention sur la structure polymérique, oligomérique ou dendritique peut être statistique, en position terminale ou sous forme de blocs.

Dans le cadre de l'invention, les monomères cyanoacrylates sont des monomères capables de polymériser par voie anionique en présence d'un agent nucléophile. Par polymérisation anionique, on entend le mécanisme défini dans l'ouvrage "Advanced Organic Chemistry", Third Edition de Jerry March, pages 151 à 161.

Les monomères cyanoacrylate de formule (I) selon la présente invention peut être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

Les compositions mises en oeuvre conformément à l'invention ont généralement une concentration en monomère cyanoacrylate selon l'invention allant de 0,1 à 80 %, de préférence de 1 à 50% en poids par rapport au poids total de la composition, plus particulièrement de 1 à 20 % en poids par rapport au poids total de la composition.

Les agents nucléophiles susceptibles d'initier la polymérisation anionique sont des systèmes connus en eux-mêmes, capables de générer un carbanion au contact d'un agent nucléophile, tels que les ions hydroxyles contenus dans l'eau. On entend par " carbanion ", les espèces chimiques définies dans " Advanced Organic Chemistry, Third Edition ", de Jerry March, page 141.

Les agents nucléophiles peuvent être peut être appliqués indépendamment de la composition de l'invention. Il peut aussi être ajouté à la composition de l'invention au moment de l'emploi.

L'agent nucléophile est un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PHNH⁻, pyridine, ArS⁻, R-C^{≡}C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻, H₂O, Ph représentant un groupe phényle ; Ar représentant un groupe aryle et R représentant un groupe alkyle en C1-C10.

Les monomères cyanoacrylates peuvent être synthétisés selon les méthodes connues décrites dans la technique. En particulier, les monomères cyanoacrylates peuvent être synthétisés selon l'enseignement du US 3 527 224, US 3 591 767, US 3 667 472, US 3 995 641, US 4 035 334 et US 4 650 826.

On peut introduire dans les compositions des inhibiteurs de polymérisation, et plus particulièrement des inhibiteurs de polymérisation anioniques et/ou radicalaires, ceci afin d'accroître la stabilité de la composition dans le temps. De façon non limitative, on peut citer les inhibiteurs de polymérisation suivants : le dioxyde de soufre, l'oxyde nitrique, le trifluorure de bore, l'hydroquinone et ses dérivés tels que l'hydroquinone monéthyléther, la TBHQ, la benzoquinone et ses dérivés tels que la duroquinone, le catéchol et ses dérivés tels que le t-butyl catéchol et le méthoxycatéchol, l'anisole et ses dérivés tels que le méthoxyanisole ou l'hydroxyanisole, le pyrogallol et ses dérivés, le p-méthoxyphénol, l'hydroxybutyl toluène, les alkyl sulfates, les alkyl sulfites, les alkyl sulfones, les alkyl sulfoxydes, les alkyl sulfures, les mercaptans, le 3-sulfonène et leurs mélanges. Les groupements alkyle désignent de préférence des groupement ayant 1 à 6 atomes de carbone.

On peut aussi utiliser à titre d'inhibiteur les acides minéraux ou organiques.

Ainsi la composition cosmétique selon l'invention peut également comprendre au moins un acide minéral ou organique, ce dernier ayant un ou plusieurs groupements carboxyliques ou sulfoniques, présentant un pKa compris entre 0 et 6 tels que l'acide phosphorique, l'acide chlorhydrique, l'acide nitrique, l'acide benzène- ou toluène-sulfonique, l'acide sulfurique, l'acide carbonique, l'acide fluorhydrique, l'acide acétique, l'acide formique, l'acide propionique, l'acide benzoïque, les acides mono-, di- ou trichloroacétiques, l'acide salicylique et l'acide trifluoroacétique, l'acide octanoïque, l'acide heptanoïque et l'acide hexanoïque.

De préférence, l'acide acétique est utilisé.

La concentration en inhibiteur dans la composition cosmétique de l'invention peut être comprise entre 10 ppm et 30% en poids et plus préférentiellement entre 10 ppm et 15% en poids par rapport au poids total de la composition.

Par composé glucidique on entend au moins l'un des composés suivants :
- un monosaccharide
- un oligosaccharide
- un polymère comportant au moins un motif sucre.

Les unités saccharidiques peuvent être en outre substituées par des groupements alkyl, ou hydroxyalkyl, ou alcoxy, ou acyloxy, ou carboxyle.

Par monosaccharide, on désigne les glucides les plus simples, non hydrolysables. Ils sont de deux types : les aldoses, les cétoses et les sucres alcools. On les distingue aussi suivant le nombre d'atomes de carbone qu'ils possèdent : les trioses, les pentoses, les hexoses et les cétoses. On peut citer comme exemple, le glucose, le mannose, le fructose, le galactose, la glucosamine, la mannosamine, le xylose, le ribose, le sorbitol, l'acide glucuronique, l'acide galacturonique.

Par oligosaccharide, on désigne un enchaînement de monosaccharides comprenant de 2 à 10 unités monosaccharides unies par des liaisons glycosidiques. On peut citer comme exemple, le lactose, le saccharose, le maltose, les fructo-oligosaccharides, les dextrines.

### Polymère à motif sucre :

Par "motif sucre", on désigne au sens de la présente invention, une portion monosaccharidique (i.e. monosaccharide ou oside ou sucre simple) ou une portion oligosaccharidique (chaînes courtes formées de l'enchaînement d'unités monosaccharidiques, éventuellement différentes) ou une portion polysaccharidique [longues chaînes constituées d'unités monosaccharidiques, éventuellement différentes, i.e. polyholosides ou polyosides (homopolyosides ou hétéropolyosides)].

Selon la présente invention, le ou les polymères comportant au moins un motif sucre sont choisis parmi les polysaccharides naturels éventuellement modifiés et les polymères synthétiques comprenant au moins un motif glucidique.

Les polysaccharides sont choisis parmi les fructanes, les gellanes, les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène, les dextranes, les celluloses et leurs dérivés (méthylcelluloses, hydroxyalkylcelluloses, éthylhydroxyéthylcelluloses, carboxyméthyl-celluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénanes, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et ioniques, les gommes de biopolysaccharides d'origine microbienne telles que les gommes de scléroglucane ou de xanthane, les mucopolysacchrides et notamment les chondroïtines sulfate et leurs mélanges.

D'une manière générale, les composés de ce type, utilisables dans la présente invention, sont choisis parmi ceux qui sont notamment décrits dans "Encyclopedia of Chemical Technology, Kirk-Othmer, Third Edition, 1982, volume 3, pp. 896-900, et volume 15, pp 439-458", dans "Polymers in Nature, par E. A. MacGREGOR et C. T. GREENWOOD, Editions John Wiley & Sons, Chapter 6, pp 240-328, 1980", dans l'ouvrage de Robert L. DAVIDSON intitulé "Handbook of Water soluble gums and resins" édité chez McGraw Hill Book Company (1980) et dans l'Industrial Gums - Polysaccharides and their Derivatives, Edité par Roy L. WHISTLER, Second Edition, Edition Academic Press Inc.", le contenu de ces trois ouvrages étant totalement inclus dans la présente demande à titre de référence.

Les gommes de guar non ioniques peuvent être modifiées ou non modifiées. Les gommes de guar non modifiées sont par exemple les produits vendus sous la dénomination VIDOGUM GH 175 par la société UNIPECTINE, et sous les dénominations MEYPRO-GUAR 50 et JAGUAR C par la société MEYHALL. Les gommes de guar non-ioniques modifiées sont notamment modifiées par des groupements hydroxyalkyle en C1-C 6. Parmi les groupements hydroxyalkyle, on peut mentionner à titre d'exemple, les groupements hydroxyméthyle, hydroxyéthyle, hydroxypropyle et hydroxybutyle.

Ces gommes de guar sont bien connues de l'état de la technique et peuvent, par exemple être préparées en faisant réagir des oxydes d'alcènes correspondants, tels que par exemple des oxydes de propylène, avec la gomme de guar de façon à obtenir une gomme de guar modifiée par des groupements hydroxypropyle. Le taux d'hydroxyalkylation, qui correspond au nombre de molécules d'oxyde d'alkylène consommées par le nombre de fonctions hydroxyle libres présentes sur la gomme de guar, varie de préférence de 0,4 à 1,2.

De telles gommes de guar non-ioniques éventuellement modifiées par des groupements hydroxyalkyle sont par exemple vendues sous les dénominations commerciales JAGUAR HP8, JAGUAR HP60 et JAGUAR HP120, JAGUAR DC
293 et JAGUAR HP 105 par la société RHONE POULENC (MEYHALL) ou sous
la dénomination GALACTASOL 4H4FD2 par la société AQUALON.

Les gommes de guar anioniques sont des polymères comportant des groupements dérivés d'acide carboxylique, sulfonique ou phosphorique.

Les gomme de xanthanes sont représentés par exemple par les produits vendus sous les dénominations KELTROL, KELTROL T, KELTROL TF, KELTROL BT, KELTROL RD, KELTROL CG par la société NUTRASWEET KELCO, ou sous les dénominations RHODICARE S, RHODICARE H par la société RHODIA CHIMIE.

Les fructanes ou fructosanes sont des oligosaccharides ou des polysaccharidescomprenant un enchaînement d'unités anhydrofructose éventuellement associé à un plusieurs résidus saccharidiques différents du fructose. Les fructanes peuvent être linéaires ou ramifiés. Les fructanes peuvent être des produits obtenus directement à partir d'une source végétale ou microbienne ou bien des produits dont la longueur de chaîne a été modifiée (augmentée ou réduite) par fractionnement, synthèse ou hydrolyse en particulier enzymatique. Les fructanes ont généralement un degré de polymérisation de 2 à environ 1000 et de préférence de 3 à environ 60.

On distingue 3 groupes de fructanes. Le premier groupe correspond à des produits dont les unités fructose sont pour la plupart liées par des liaisons beta-2-1. Ce sont des fructanes essentiellement linéaires tes que les inulines. Le second groupe correspond également à des fructoses linéaires mais les unités fructose sont essentiellement liées par des liaisons beta-2-6. Ces produits sont des levanes.

Le troisième groupe correspond à des frucanes mixtes, c'est à dire ayant des enchaînements beta-2-6 et beta-2-1. Ce sont des fructanes essentiellement ramifiés tes que les graminanes. Les fructanes préférés sont les inulines. L'inuline peut être obtenue par exemple à partir de chicorée, de dahlia ou de topinambours.

Les polymères synthétiques à greffon sucre sont notamment choisis parmi:
- Les polydiméthylsiloxane (PDMS) à motif sucre qui peuvent être synthétisées par réaction d'une PDMS à fonction époxy avec un carbohydrate comme décrit dans la publication de R. Wagner et Col., Appl. Organometallic Chem. 1998, V12, p47, par réaction d'un aldonolactone avec une aminopropyl silicone ou par hydrosilylation d'un allyl-glycoside avec une polyhydromethylsiloxane (PHMS) comme décrit dans la pûblication de V. Rraunmühl et col, Macromolécules 1995, V28, p17.
- Les polymères à squelette acrylamide, acrylate, polyester, polyamide, vinylique à motif sucre qui peuvent être synthétisés par polyaddition, polycondensation ou polymérisation radicalaire comme décrit dans la publication de J. Kadokawa et col., Synlett., 1999, 1845-1856.

Le polysaccharide est de préférence une cellulose, une hemicellulose, une lignocellulose, un amidon, une inuline, une gomme de guar, une gomme de xanthane, un pullulane, un agar-agar, un alginate de sodium, de potassium ou d'ammonium, un carragheenane, un dextrane, un furcellarane, une gomme de gellane, une gomme arabique, une gomme adragante, un acide hyaluronique, un mannane de konjac, un sulfonate de lignine, une gomme de caroube, une chitinepartiellement N-actylée, une pectine, un polydextrose, une gomme de rhamsane ou une gomme de welane. Plus préférentiellement, le polysaccharide est une cellulose, une hémicellulose, une carboxyméthyl cellulose, une hydroxyéthyl cellulose, une hydroxypropyl cellulose, une hydroxypropylméthyl cellulose, une méthylcellulose, une lignocellulose, un amidon, un acétate d'amidon, un hydroxyéthylamidon, un hydroxypropylamidon,une inuline, une gomme de guar, une gomme de carboxyméthylguar,une gomme de carboxyméthylhydroxypropylguar, une gommed'hydroxyéthylguar, une gomme d'hydroxypropylguar ou une gomme de xanthane et leurs mélanges.

Le polysaccharide présente de préférence une masse moléculaire moyenne en poids comprise entre 500 et 15 000 000, mieux encore entre 1000 et 10 000 000.

Le ou les composés glucidiques représentent généralement de 0,01 à 80 %, de préférence de 0,1 à 40 %, en poids par rapport au poids total de la composition, plus particulièrement de 0,5 à 10% en poids.

Les compositions conformes à l'invention peuvent également contenir au moins un pigment. Cela permet d'obtenir une coloration visible sur tout type de fond sans décoloration au préalable. Cette coloration est rémanente aux shampooings.

Les pigments utiles dans la présente invention sont choisis parmi tous les pigments organiques et / ou minéraux connus de la technique, notamment ceux qui sont décrits dans l'encyclopédie de technologie chimique de Kirk-Othmer et dans l'encyclopédie de chimie industrielle de Ullmann.

Ces pigments peuvent se présenter sous forme de poudre ou de pâte pigmentaire. Ils peuvent être enrobés ou non enrobés.

Les pigments conformes à l'invention peuvent par exemple être choisis parmi les pigments blancs ou colorés, les laques, les pigments à effets spéciaux tels que les nacres ou les paillettes, et leurs mélanges.

A titre d'exemples de pigments minéraux blancs ou colorés, on peut citer le dioxyde de titane, traité ou non traité en surface, les oxydes de zirconium ou de cérium, les oxydes de fer ou de chrome, le violet de manganèse, le bleu outremer, l'hydrate de chrome et le bleu ferrique. Par exemple, les pigments minéraux suivants peuvent être utilisés : Ta₂O₅, Ti₃O₅, Ti₂O₃, TiO, ZrO₂ en mélange avec TiO₂, ZrO₂, Nb₂O₅, CeO₂, ZnS.

A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références Cl 42090, 69800, 69825, 73000, 74100, 74160, les pigments jaunes codifiés dans le Color Index sous les références Cl 11680, 11710, 15985, 19140, 20040, 21100, 21108, 47000, 47005, les pigments verts codifiés dans le Color Index sous les références Cl 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom :
- JAUNE COSMENYL IOG : Pigment YELLOW 3 (CI 11710) ;
- JAUNE COSMENYL G : Pigment YELLOW 1 (CI 11680) ;
- ORANGE COSMENYL GR : Pigment ORANGE 43 (CI 71105) ;
- ROUGE COSMENYL R : Pigment RED 4 (CI 12085) ;
- CARMIN COSMENYL FB : Pigment RED 5 (CI 12490) ;
- VIOLET COSMENYL RL : Pigment VIOLET 23 (CI 51319) ;
- BLEU COSMENYL A2R : Pigment BLUE 15.1 (CI 74160) ;
- VERT COSMENYL GG : Pigment GREEN 7 (CI 74260) ;
- NOIR COSMENYL R : Pigment BLACK 7 (CI 77266).

Les pigments conformes à l'invention peuvent aussi être sous forme de pigments composites tels qu'ils sont décrits dans le brevet EP 1 184 426. Ces pigments composites peuvent être composés notamment de particules comportant un noyau inorganique, au moins un liant assurant la fixation des pigments organiques sur le noyau, et au moins un pigment organique recouvrant au moins partiellement le noyau.

Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille.

A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 1 O (CI 77 002), D & C Green 3 (CI 42 053), D & C Blue 1 (CI 42 090).

Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semi-transparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. A titre de pigments nacrés, on peut citer les nacres Cellini commercialisée par Engelhard (Mica-TiO2-laque), Prestige commercialisée par Eckart (Mica-TiO2), Colorona commercialisée par Merck (Mica-TiO2-Fe2O3).

On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation.

Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriqués selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of phisical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

La variété des pigments qui peuvent être utilisés dans la présente invention permet d'obtenir une riche palette de couleurs, ainsi que des effets optiques particuliers tels que des effets métalliques, interférentiels.

Selon un mode de réalisation particulier, les pigments sont des pigments colorés. On entend par pigment coloré des pigments autres que les pigments blancs.

La taille du pigment utile dans le cadre de la présente invention est généralement comprise entre 10 nm et 200 µm, de préférence entre 20 nm et 80 µm, et plus préférentiellement entre 30 nm et 50 µm.

Le ou les pigments sont chacun généralement présents dans la composition conforme à l'invention dans des quantités généralement comprises entre 0,05 et 50 % du poids total de la composition, de préférence de 0,1 à 35 %.

Les compositions selon l'invention peuvent comprendre un gélifiant/structurant des milieux non aqueux comme les polyamides siliconés PSPA (DP100 et DP15) commercialisés par Dow Chemical, les KSG organiques polylauryldiméthylsiloxane et les KSG siliconés commercialisés par Shinetsu, le palmitate de dextrine et le stéarate d'inuline (gamme Rheopearl de Chiba Flour Milling) ; les acrylate à chaîne alkyle commercialisé par Landec, les copolymères éthylène octène commercialisé par Dupont de Nemours, le dibutyl lauryl glutamide commercialisé par Ajinomoto, le disorbène commercialisé par Roquette, les copolymères styrène acrylate Versagel M5960 et 5670 commercialisés par Penreco, les kratons dibloc et tribloc commercialisés par Kraton Polymers, l'acide hydroxystéariques, les cires de jojoba, les silicices pyrogénées commercialisées par Degussa, les cires de silicones commercialisés par Waker, le polyamide Uniclear commercialisé par Arizona Chemical, la bentone.

Les compositions selon l'invention peuvent comprendre un propulseur. Le propulseur est constitué par les gaz comprimés ou liquéfiés usuellement employés pour la préparation de compositions aérosols. On emploiera de manière préférentielle l'air, le gaz carbonique, l'azote comprimé ou encore un gaz soluble tel que le diméthyléther, les hydrocarbures halogénés (fluorés en particuliers) ou non (butane, propane, isobutane) et leurs mélanges.

Les compositions conformes à l'invention peuvent également contenir au moins un agent utilisé habituellement en cosmétique, choisi, par exemple, parmi des agents réducteurs, des corps gras, des plastifiants, des adoucissants, des agents anti-mousse, des agents hydratants, des pigments, des argiles, des charges minérales, des filtres UV, des colloïdes minéraux, des peptisants, des solubilisants, des parfums, des conservateurs, des tensio-actifs anioniques, cationiques, non ioniques ou amphotères, des polymères fixants ou non, des polyols non glucosidiques, des protéines, des vitamines, des colorants directs ou d'oxydation, des agents nacrants, des propulseurs, et des épaississants minéraux ou organiques et les N-acylaminoacides, les cires oxyéthylénées ou non, les paraffines, les acides gras en C10-C30 tels que l'acide stéarique, l'acide laurique, les amides gras en C10-C30 tel que le diéthanolamide laurique, les esters solides d'alcool gras en C10-C30 et leurs mélanges.

Ces agents ou le(s) composé(s) glucidique(s) peuvent être éventuellement encapsulés. La capsule peut être de type polycyanoacrylate.

La composition est utilisée de préférence sur les fibres kératiniques comme les cheveux, de préférence en présence d'un agent nucléophile, pour leur traitement cosmétique.

Elle est notamment utilisée pour conférer un effet de conditionnement tel que de la douceur ; du démêlage; de la brillance ; du volume et/ou un effet coiffant comme le maintien de la chevelure.

Un procédé de traitement cosmétique selon l'invention comprend l'application d'une composition telle que définie ci-dessus, sur les fibres kératiniques comme les cheveux, de préférence en présence d'un agent nucléophile tel que défini ci-dessous.

Les agents nucléophiles susceptibles d'initier la polymérisation anionique sont des systèmes connus en eux-mêmes, capables de générer un carbanion au contact d'un agent nucléophile, tels que les ions hydroxydes contenus dans l'eau à pH neutre. On entend par « carbanion », les espèces chimiques définies dans « Advanced Organic Chemistry, Third Edition », de Jerry March, page 141.

Les agents nucléophiles peuvent être constitués par un composé moléculaire, un oligomère, un dendrimère ou un polymère possédant des fonctions nucléophiles. De façon non limitative, on peut citer comme fonctions nucléophiles les fonctions : R₂N⁻, NH₂⁻, Ph₃C⁻, R₃C⁻, PHNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

De préférence, l'agent nucléophile est l'eau. Cette eau peut être apportée par une humidification préalable.

II est également possible, afin de moduler la cinétique de réaction, de préalablement humidifier les fibres kératiniques comme les cheveux, à l'aide d'une solution aqueuse dont le pH a été ajusté à l'aide d'une base, d'un acide ou d'un mélange acide/base. L'acide et/ou la base peuvent être inorganiques ou organiques.

Il est également possible de moduler la cinétique de polymérisation par voie anionique en pré-imprégnant les fibres kératiniques comme les cheveux, à l'aide d'un agent nucléophile autre que l'eau. L'agent nucléophile peut être utilisé pur, en solution, sous forme d'une émulsion ou être encapsulé.

Pour moduler la cinétique de polymérisation anionique, on peut également augmenter la nucléophilie des fibres kératiniques comme les cheveux, par transformation chimique de la matière kératinique.

A titre d'exemple de transformation chimique, on peut citer la réduction des ponts di-sulfure composant en partie la kératine en thiols avant application de la composition de l'invention. De façon non exhaustive, on peut citer comme réducteurs des ponts disulfure composant en partie la kératine, les composés suivants:
- thiosulfate de sodium anhydre, métabisulfite de sodium en poudre, thiourée, sulfite d'ammonium, acide thioglycolique, acide thiolactique, thiolactate d'ammonium, mono-thioglycolate de glycérol, thioglycolate d'ammonium, thioglycérol, acide 2,5-dihydroxybenzoique, di-thioglycolate de diammonium, thioglycolate de strontium, thioglycolate de calcium, formosulfoxylate de zinc, thioglycolate d'isooctyle, dl-cystéine, thioglycolate de monoéthanolamine.

Pour moduler la cinétique de polymérisation par voie anionique, et plus précisément réduire la vitesse de polymérisation des monomères de l'invention, il est possible d'augmenter la viscosité de la composition. Pour ce faire, on peut ajouter à la composition de l'invention un ou des polymères ne présentant pas de réactivité sur les monomères conformes à l'invention. Dans ce cadre, on peut citer de façon non exhaustive le poly(méthacrylate de méthyle) (PMMA) ou encore les copolymères à base de cyanoacrylate tels qu'ils sont décrits dans le brevet US 6,224,622.

Afin d'améliorer entre autres l'adhésion du poly(cyanoacrylate) formé in situ, on peut pré-traiter la fibre avec tous types de polymères, ou réaliser un traitement capillaire avant application de la composition de l'invention, comme une coloration directe ou d'oxydation, une permanente ou encore un défrisage.

L'application des compositions telles que décrites ci-dessus peut être suivie ou non d'un rinçage.

Elles peuvent se présenter sous la forme de lotion, de spray, de mousse.

La composition conforme à l'invention peut se présenter telle quelle ou résulter du mélange au moment de l'emploi de deux compositions. Par exemple, la première composition peut comprendre le ou les monomères cyanoacrylates et la deuxième composition peut comprendre le ou les composés glucidiques.

La composition comprenant le ou les monomères cyanoacrylates est anhydre. La composition comprenant le ou les composés glucidiques peut être anhydre, aqueuse, alcoolique et éventuellement se présenter sous forme d'émulsions eau-dans-huile ou huile-dans-eau. Lorsque le composé glucidique se présente sous forme de solutions ou dispersions aqueuse ou alcoolique (notamment éthanolique), la composition le contenant est généralement aqueuse ou alcoolique sous forme d'émulsions eau-dans-huile ou huile-dans-eau.

Le procédé selon la présente invention comprend l'application sur les fibres kératiniques d'une composition comprenant, dans un milieu cosmétiquement acceptable, au moins un composé glucidique et au moins un monomère cyanoacrylate telle que définie précédemment en présence d'au moins un agent nucléophile.

Selon une variante du procédé de l'invention, l'application de la composition conforme à l'invention est réalisée en au moins deux étapes, une des étapes consiste à appliquer sur les fibres kératiniques une composition comprenant le ou les composés glucidiques et avec ou sans rinçage intermédiaire, une autre étape consistant à appliquer sur les fibres kératiniques une composition comprenant le ou les monomères acrylates, l'ordre des étapes étant indifférent.

Un autre mode de réalisation particulier de l'invention consiste en ce que l'application d'au moins un monomère cyanoacrylate se fait avant l'application d'au moins un composé glucidique.

Un mode de réalisation particulier de l'invention consiste en ce que l'application d'au moins un composé glucidique se fait avant l'application d'au moins un monomère cyanoacrylate.

Un mode de réalisation préféré de l'invention consiste en un procédé comprenant les étapes consistant à :
(1) appliquer sur les cheveux une composition comprenant de 0,1 à 59,9 % en poids, et mieux encore 0,25-25 % en poids d'un composé glucidique, les pourcentages étant exprimés par rapport au poids total de la solution ou de la dispersion,
(2) appliquer sur les cheveux, après un éventuel rinçage intermédiaire, une composition comprenant 0,1-40 % en poids, de préférence de 1 à 20 % en poids de monomère(s) cyanoacrylate(s) tel(s) que défini(s) ci-dessus, les pourcentages étant exprimés par rapport au poids total de la composition.

L'ordre des deux étapes (1) et (2) peut être inversé.

L'application d'un produit cosmétique peut en outre précéder la première étape ou suivre la deuxième étape. Chaque étape peut être également interrompue par un rinçage et éventuellement par un séchage, le séchage pouvant être réalisé au moyen d'un casque, d'un sèche-cheveux et/ou d'un fer plat ou à friser.

Les étapes des procédés décrits ci-dessus peuvent être répétées de manière à obtenir plusieurs couches selon le type de dépôt que l'on recherche en termes de nature chimique, résistance mécanique, épaisseur, aspect et/ou toucher.

L'invention a encore pour objet un kit comprenant une première composition contenant au moins un monomère cyanoacrylate tel que défini ci-dessus et éventuellement au moins un inhibiteur de polymérisation anionique et/ou radicalaire tel que défini ci-dessus, ainsi qu'une deuxième composition comprenant dans un milieu cosmétiquement acceptable au moins un composé glucidique et éventuellement une troisième composition qui contient l'agent nucléophile.

La présente invention a aussi pour objet un kit (pour le traitement des fibres kératiniques notamment les cheveux), comprenant une composition comprenant au moins un composé glucidique tel que défini précédemment et une deuxième composition comprenant au moins un monomère cyanoacrylate tel que défini précédemment et éventuellement au moins un inhibiteur de polymérisation.

L'invention a encore pour objet un kit comprenant une première composition contenant au moins un monomère cyanoacrylate tel que défini ci-dessus et un solvant organique liquide, et éventuellement au moins un inhibiteur de polymérisation anionique et/ou radicalaire et une deuxième composition comprenant dans un milieu cosmétiquement acceptable au moins un composé glucidique.

Selon un mode de réalisation particulier du kit conforme à l'invention, la composition comprenant le ou les composés glucidiques comprend de plus au moins un agent nucléophile tel que défini précédemment.

Selon un autre mode de réalisation particulier du kit conforme à l'invention, le kit contient de plus une composition comprenant au moins un agent nucléophile tel que défini précédemment.

Selon une première variante de l'invention, la composition comprenant le ou les composés glucidiques et la composition comprenant le ou les monomères cyanoacrylates et éventuellement le ou les inhibiteurs de polymérisation sont présents dans une seule composition anhydre.

Selon une deuxième variante, la composition comprenant le ou les composés glucidiques est une composition aqueuse et la composition comprenant le ou les monomères cyanoacrylates et éventuellement le ou les inhibiteurs de polymérisation est une composition anhydre. Dans ce cas, les compositions sont mélangées au moment de l'emploi ou appliquées successivement

Le ou les monomères cyanoacrylates sont présents dans la composition les comprenant dans une concentration généralement comprise entre 5 et 80 % en poids environ du poids total de la composition, de préférence entre 1 et 30 % en poids, et encore plus préférentiellement entre 0,1 et 20 % en poids.

Les exemples suivants sont donnés à titre illustratif de la présente invention.

Dans les exemples suivants, toutes les quantités sont indiquées en pour cent en poids de matière active par rapport au poids total de la composition, sauf indication contraire.

### EXEMPLES

### Exemple 1 :

La composition A suivante est réalisée :

| | |
|---|---|
| Hydroxyéthylcellulose | 1,5g |
| Eau | 98,5g |

La composition B suivante est réalisée :

| | |
|---|---|
| Poly diméthyl siloxane alpha-omega dihydroxylé / cyclopentadiméthylsiloxane (14,7 / 85,3 % en poids) commercialisé par Dow Corning sous le nom de DC 1501 Fluid | 45g |
| Cyclopentadiméthylsiloxane commercialisé par Dow Corning sous le nom de DC245 Fluid | 44,75g |
| Acide acétique | 0.25g |
| Méthylheptylcyanoacrylate de Chemence | 10g |

0.3g de la composition A est appliqué sur une mèche de cheveux de 1g préalablement shampooinée. Ensuite, 0.5g de la composition B est appliquée. Après 15 min de pause à température ambiante, la mèche est séchée au sèche-cheveux pendant 3min.

Les cheveux sont gainés, texturisés au toucher. Cet effet est encore visible après plusieurs shampooings.

Les cheveux ne deviennent pas gras au cours du temps contrairement aux cheveux traités uniquement avec de l'eau à la place de la composition A. Les cheveux ont un aspect propre.

### Exemple 2 :

La composition A suivante est réalisée :

| | |
|---|---|
| Carboxyméthyl Hydroxypropyl guar | 1,5g |
| Eau | 98,5g |

La composition B suivante est réalisée :

| | |
|---|---|
| Poly diméthyl siloxane alpha-omega dihydroxylé / Cyclopentadiméthylsiloxane (14,7 %/ 85,3% en poids) commercialisé par Dow Corning sous le nom de DC 1501 Fluid | 45g |
| Cyclopentadiméthylsiloxane commercialisé par Dow Corning sous le nom de DC245 Fluid | 44,75g |
| Acide acétique | 0.25g |
| Méthylheptylcyanoacrylate de Chemence | 10g |

0.3g de la composition A est appliqué sur une mèche de cheveux de 1g préalablement lavée avec un shampooing. Ensuite, 0.5g de la composition B est appliquée. Après 15 min de pause à température ambiante, la mèche est séchée au sèche-cheveux pendant 3min. Les cheveux sont gainés, texturisés au toucher. Cet effet est encore visible après plusieurs shampooings.

Les cheveux ne deviennent pas gras au cours du temps contrairement aux cheveux traités uniquement avec de l'eau à la place de la composition A. Les cheveux ont un aspect propre.

### Exemple 3 :

La composition A suivante est réalisée :

| | A |
|---|---|
| Chondroitine sulfate de sodium* | 1,5g |
| Eau | 98,5g |

| | |
|---|---|
| **Commercialisé par Sederma sous le nom d'Hydromarine* | |

La composition B suivante est réalisée :

| | |
|---|---|
| Poly diméthyl siloxane alpha-omega dihydroxylé / Cyclopentadiméthylsiloxane (14,7 / 85,3) commercialisé par Dow Corning sous le nom de DC 1501 Fluid | 45g |
| Cyclopentadiméthylsiloxane commercialisé par Dow Corning sous le nom de DC245 Fluid | 44,75g |
| Acide acétique | 0.25g |
| Méthylheptylcyanoacrylate de Chemence | 10g |

0.3g de la composition A est appliqué sur une mèche de cheveux de 1g préalablement lavée avec un shampooing. Ensuite, 0.5g de la composition B est appliquée. Après 15 min de pause à température ambiante, la mèche est séchée au sèche-cheveux pendant 3min.

Les cheveux sont gainés, texturisés au toucher. Cet effet est encore visible après plusieurs shampooings.

Les cheveux ne deviennent pas gras au cours du temps contrairement aux cheveux traités uniquement avec de l'eau à la place de la composition A. Les cheveux ont un aspect propre.

## Revendications

1. Composition cosmétique comprenant au moins un monomère cyanoacrylate, au moins un composé glucidique et au moins un solvant organique liquide,
le monomère cyanoacrylate étant choisi parmi les monomères de formule (I): 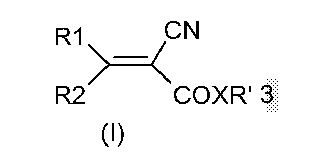 dans laquelle :
X désigne NH, S ou O,
R1 et R2 désignent chacun un atome d'hydrogène,
R'3 représentant un atome d'hydrogène ou un groupe hydrocarboné saturé ou non, linéaire, ramifié ou cyclique, comportant de préférence de 1 à 20, mieux encore de 1 à 10 atomes de carbone, et contenant éventuellement un ou plusieurs atomes d'azote, d'oxygène, de soufre, et éventuellement substitué par un ou plusieurs groupements choisis parmi -OR', -COOR', - COR', -SH, -SR', -OH, les atomes d'halogène, ou un résidu de polymère pouvant être obtenu par polymérisation radicalaire, par polycondensation ou par ouverture de cycle, R' désignant un groupe alkyle en C₁-C₁₀.

2. Composition cosmétique selon la revendication 1, **caractérisée en ce que** le monomère cyanoacrylate est choisi parmi les monomères de formule (IV): 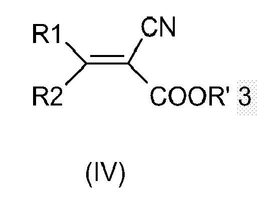
R1 et R2 désignent chacun un atome d'hydrogène,
dans laquelle R'3 représente un radical alkyle en C1-C10, alcényle en C2-C10 ou alcoxy(C1-C4) alkyle(C1-C10).

3. Composition cosmétique selon la revendication 2, **caractérisée en ce que** le monomère cyanoacrylate est choisi parmi le 2-cyanoacrylate d'éthyle, le 2-cyanoacrylate de méthyle, le 2-cyanoacrylate de n-propyle, le 2-cyanoacrylate d'isopropyle, le 2-cyanoacrylate de tert-butyle, le 2-cyanoacrylate de n-butyle, le 2-cyanoacrylate d'iso-butyle, le cyanoacrylate de 3-méthoxybutyle, le cyanoacrylate de n-décyle, le 2-cyanoacrylate d'hexyle, le 2-cyanoacrylate de 2-éthoxyéthyle, le 2-cyanoacrylate de 2-méthoxyéthyle, le 2-cyanoacrylate de methoxypropyle, le 2-cyanoacrylate de 2-octyle, le 2-cyanoacrylate de 2-propoxyéthyle, le 2-cyanoacrylate de n-octyle le 2-cyanoacrylate d'allyle, le 2-cyanoacrylate de méthoxypropyle et le cyanoacrylate d'iso-amyle.

4. Composition cosmétique selon l'une quelconques des revendications 3 ou 4, **caractérisée en ce que** le monomère cyanoacrylate est choisi parmi les cyanoacrylates d'alkyle en C6-C10.

5. Composition cosmétique selon la revendication 4, **caractérisée en ce que** le monomère cyanoacrylate est choisi parmi les monomères les cyanoacrylates d'octyle de formule (V) et leurs mélanges : 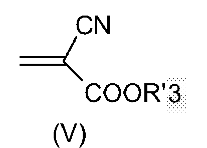 dans laquelle :
R'3 = -(CH2)₇-CH₃,
-CH(CH₃)-(CH₂)₅-CH₃,
-CH₂-CH(C₂H₅)-(CH₂)₃-CH₃,
-(CH₂)₅-CH(CH₃)-CH₃,
-(CH₂)₄-CH(C₂H₅)-CH₃.

6. Composition cosmétique selon l'une quelconque des revendications précédentes, **caractérisé en ce que** le ou les monomères cyanoacrylate sont fixés de façon covalente sur des supports tels que des polymères, des oligomères ou des dendrimères

7. Composition selon l'une quelconque des revendications précédentes, **caractérisée en ce que** la quantité de monomère cyanoacrylate va de 0,1 à 80 % en poids, par rapport au poids total de la composition.

8. Composition selon l'une quelconque des revendications 1 à 7, **caractérisée par le fait que** le composé glucidique est choisis parmi :
• les monosaccharides
• les oligosaccharides
• les polymères comportant au moins un motif sucre.

9. Composition selon l'une quelconque des revendications 1 à 8, **caractérisée par le fait que** le composé glucidique est choisi parmi le glucose, le mannose, le fructose, le galactose, la glucosamine, la mannosamine, le xylose, le ribose, le sorbitol, l'acide glucuronique, l'acide galacturonique

10. Composition selon l'une quelconque des revendications 1 à 8, **caractérisée par le fait que** le composé glucidique est choisi parmi le lactose, le saccharose, le maltose, les fructo-oligosaccharides, les dextrines.

11. Composition selon la revendication 8, **caractérisée par le fait que** le polymère à motif sucre est choisi parmi les polysaccharides naturels éventuellement modifiés et les polymères synthétiques comprenant au moins un motif glucidique.

12. Composition selon la revendication 11, **caractérisée par le fait que** les polysaccharides sont choisis parmi les fructanes, les gellanes, les glucanes, les amidons modifiés ou non (tels que ceux issus, par exemple, de céréales comme le blé, le maïs ou le riz, de légumes comme le pois blond, de tubercules comme les pommes de terre ou le manioc), l'amylose, l'amylopectine, le glycogène les dextranes, les celluloses et leurs dérivés (méthylcelluloses, hydroxyalkylcelluloses, éthylhydroxyéthylcelluloses, carboxyméthyl-celluloses), les mannanes, les xylanes, les lignines, les arabanes, les galactanes, les galacturonanes, la chitine, les chitosanes, les glucoronoxylanes, les arabinoxylanes, les xyloglucanes, les glucomannanes, les acides pectiques et les pectines, l'acide alginique et les alginates, les arabinogalactanes, les carraghénanes, les agars, les glycosaminoglucanes, les gommes arabiques, les gommes Tragacanthe, les gommes Ghatti, les gommes Karaya, les gommes de caroube, les galactomannanes telles que les gommes de guar et leurs dérivés non ioniques (hydroxypropyl guar) et ioniques, les gommes de biopolysaccharides d'origine microbienne telles que les gommes de scléroglucane ou de xanthane, les mucopolysacchrides et notamment les chondroïtines sulfate et leurs mélanges.

13. Composition selon la revendication 12, **caractérisée par le fait que** les polysaccharides sont choisis une cellulose, une hémicellulose, une carboxyméthyl cellulose, une hydroxyéthyl cellulose, une hydroxypropyl cellulose, une hydroxypropylméthyl cellulose, une méthylcellulose, une lignocellulose, un amidon, un acétate d'amidon, un hydroxyéthylamidon, un hydroxypropylamidon,une inuline, une gomme de guar, une gomme de carboxyméthylguar,une gomme de carboxyméthylhydroxypropylguar, une gommed'hydroxyéthylguar, une gomme d'hydroxypropylguar ou une gomme de xanthane et leurs mélanges.

14. Composition selon l'une quelconque des revendications 1 à 13, **caractérisée en ce que** le ou les composés glucidiques représentent de 0,001 % à 80 % en poids, de préférence de 0,01 % à 50% en poids, par rapport au poids total de la composition finale.

15. Composition selon l'une quelconque des revendications précédentes, **caractérisée en ce que** la composition est anhydre.

16. Composition selon l'une quelconque des revendications précédentes, **caractérisée en ce que** le solvant organique est choisi parmi les alcools aromatiques tels que l'alcool benzylique ; les alcools gras liquides , notamment en C₁₀-C₃₀; les polyols modifiés ou non tels que le glycérol, le glycol, le propylène glycol, le dipropylène glycol, le butylène glycol, le butyle diglycol ; les silicones volatiles telles que la cylopentasiloxane, la cyclohexasiloxane, les polydiméthylsiloxanes modifiées ou non par des fonctions alkyle et/ou amine et/ou imine et/ou fluoroalkyl et/ou carboxylique et/ou betaïne et/ou ammonium quaternaire, les polydiméthylsiloxanes modifiées liquides, les huiles minérales, organiques ou végétales, les alcanes et plus particulièrement les alcanes de C₅ à C_{10;} les acides gras liquides, les esters gras liquides et plus particulièrement les benzoates ou les salicylates d'alcool gras et leurs mélanges.

17. Composition selon l'une quelconque des revendications précédentes, **caractérisée en ce que** le solvant organique représente de 0,01 à 99 %, de préférence de 0,1 à 50 %, en poids par rapport au poids total de la composition.

18. Composition selon l'une quelconque des revendications précédentes, **caractérisée en ce que** la composition contient des inhibiteurs de polymérisation.

19. Composition selon la revendication 18, **caractérisée en ce que** les inhibiteurs sont des inhibiteurs de polymérisation anioniques et/ou radicalaires.

20. Composition selon la revendication 18 ou 19, **caractérisée en ce que** les inhibiteurs de polymérisation sont choisis parmi le dioxyde de soufre, l'oxyde nitrique, la lactone, le trifluorure de bore, l'hydroquinone et ses dérivés tels que le monoéthyléther d'hydroquinone, la tert-butylhydroquinone (TBHQ), la benzoquinone et ses dérivés tels que la duroquinone, le catéchol et ses dérivés tels que le t-butylcatéchol et le méthoxycatéchol, l'anisole et ses dérivés tels que le méthoxyanisole, l'hydroxyanisole ou le butylhydroxyanisole, le pyrogallol, le 2,4-dinitrophénol, le 2,4,6-trihydroxybenzène, le p-méthoxyphénol, l'hydroxybutyltoluène, les sulfates d'alkyle, les sulfites d'alkyle, les alkyl sulfones, les alkyl sulfoxydes, les alkyl sulfures, les mercaptans, le 3-sulfonène, les acides minéraux et organiques et leurs mélanges.

21. Composition selon l'une quelconque des revendications 18 à 20, **caractérisée en ce que** le ou les inhibiteurs de polymérisation sont présents en une quantité allant de 10 ppm à 20 % par rapport au poids total de la composition.

22. Composition selon l'une quelconque des revendications précédentes, **caractérisée en ce que** la composition contient en outre au moins un agent choisi parmi les agents réducteurs, les corps gras, les plastifiants, les adoucissants, les agents anti-mousse, les agents hydratants, les pigments, les argiles, les charges minérales, les filtres UV, les colloïdes minéraux, les peptisants, les solubilisants, les parfums, les conservateurs, les tensio-actifs anioniques, cationiques, non ioniques ou amphotères, les polymères fixants ou non, les polyols non glucidiques, les protéines, les vitamines, les colorants directs ou d'oxydation, les agents nacrants, les propulseurs, et les épaississants minéraux ou organiques tels que les N-acylaminoacides, les cires oxyéthylénées ou non, les paraffines, les acides gras en C10-C30 tels que l'acide stéarique, l'acide laurique, les amides gras en C10-C30 tel que le diéthanolamide laurique, les esters solides d'alcool gras en C10-C30 et leurs mélanges.

23. Utilisation de la composition selon l'une quelconque des revendications précédentes, pour le traitement cosmétique des fibres kératiniques.

24. Utilisation selon la revendication 23, pour conférer de la douceur, du démêlage, de la brillance, du volume et/ou du maintien aux cheveux.

25. Utilisation selon la revendication 23 ou 24, en présence d'un agent nucléophile.

26. Utilisation selon la revendication 25, **caractérisée en ce que** l'agent nucléophile est choisi parmi les composés moléculaires, les oligomères, les dendrimères ou polymères possédant des fonctions nucléophiles choisies parmi : R₂N⁻, NH₂⁻, Ph₃C⁻, R₃C⁻, PHNH⁻, pyridine, ArS⁻, R-C≡C⁻, RS⁻, SH, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle ; Ar représentant un groupe aryle et R représentant un groupe alkyle en C₁-C₁₀.

27. Utilisation selon la revendication 25, **caractérisée en ce que** l'agent nucléophile est l'eau.

28. Procédé de traitement cosmétique des fibres kératiniques, comprenant l'application d'une composition selon l'une quelconque des revendications précédentes 1 à 22, sur les fibres kératiniques en présence d'un agent nucléophile.

29. Procédé selon la revendication 28, **caractérisé en ce que** l'agent nucléophile est choisi parmi les composés moléculaires, les oligomères, les dendrimères ou polymères possédant des fonctions nucléophiles choisies parmi: R₂N⁻, NH₂⁻, Ph₃C⁻, R₃C⁻, PHNH⁻, pyridine, ArS⁻, R-C≡C⁻, RS⁻, SH⁻, RO⁻, R₂NH, ArO⁻, N₃⁻, OH⁻, ArNH₂, NH₃, I⁻, Br⁻, Cl⁻, RCOO⁻, SCN⁻, ROH, RSH, NCO⁻, CN⁻, NO₃⁻, ClO₄⁻ et H₂O, Ph représentant le groupe phényle, Ar représentant un groupe aryle et R représentant un groupe aryle en C₁-C₁₀.

30. Procédé selon la revendication 28, **caractérisé en ce que** l'agent nucléophile est l'eau.

31. Procédé selon l'une quelconque des revendications 28 à 30, **caractérisé en ce que** l'on applique la composition sur les fibres kératiniques préalablement humidifiées à l'aide d'une solution aqueuse dont le pH a été ajusté à l'aide d'une base, d'un acide ou d'un mélange acide/base.

32. Procédé selon l'une quelconque des revendications 28 à 30, **caractérisé en ce que** les fibres kératiniques sont pré-imprégnées à l'aide d'un agent nucléophile différent de l'eau.

33. Procédé selon l'une quelconque des revendications 28 à 32, **caractérisé en ce que** les fibres kératiniques sont préalablement réduites avant application de la composition.

34. Procédé selon l'une des revendications 28 à 33, **caractérisé en ce que** l'application de la composition est suivie d'un rinçage.

35. Procédé selon l'une des revendications 28 à 34, **caractérisé en ce que** les fibres kératiniques sont les cheveux.

36. Procédé de traitement cosmétique des cheveux, comprenant au moins deux étapes, une étape comprenant l'application d'au moins un composé glucidique et au moins un solvant organique liquide, et avec ou sans rinçage intermédiaire, une autre étape comprenant l'application d'au moins un monomère cyanoacrylate selon l'une des revendications 1 à 6.

37. Procédé selon la revendication 36, **caractérisé en ce que** l'application d'au moins un composé glucidique se fait avant l'application d'au moins un monomère cyanoacrylate selon l'une des revendications 1 à 6.

38. Procédé selon la revendication 36, **caractérisé en ce que** l'application d'au moins un monomère cyanoacrylate selon l'une des revendications 1 à 6 se fait avant l'application d'au moins un composé glucidique.

39. Procédé selon la revendication 36, **caractérisé en ce qu'**il comprend les étapes consistant à :
(1) appliquer sur les cheveux une composition comprenant de 0,1 à 59,9 % en poids, de composé glucidique, les pourcentages étant exprimés par rapport au poids total de la solution,
(2) appliquer sur les cheveux, après un éventuel rinçage intermédiaire, une composition comprenant de 0,1 à 40 % en poids, de préférence 1 à 20 % en poids, de monomère(s) cyanoacrylate(s) selon l'une des revendications 1 à 6, les pourcentages étant exprimés par rapport au poids total de la composition.

40. Kit comprenant une première composition comprenant au moins un monomère cyanoacrylate tel que défini dans les revendications 1 à 6, et éventuellement au moins un inhibiteur de polymérisation anionique et/ou radicalaire, une deuxième composition contenant un solvant organique ainsi qu'une troisième composition comprenant dans un milieu cosmétiquement acceptable au moins un composé glucidique.

41. Kit comprenant une première composition comprenant au moins un monomère cyanoacrylate tel que défini dans les revendications 1 à 6 et un solvant organique liquide, et éventuellement au moins un inhibiteur de polymérisation anionique et/ou radicalaire, une deuxième composition comprenant dans un milieu cosmétiquement acceptable au moins un composé glucidique.
